(19) **Europäisches Patentamt**
**European Patent Office**
**Office européen des brevets**

(11) **EP 1 988 507 B1**

(12) **EUROPEAN PATENT SPECIFICATION**

(45) Date of publication and mention
of the grant of the patent:
**22.09.2010 Bulletin 2010/38**

(51) Int Cl.:
**G06T 7/00** (2006.01)

(21) Application number: **08251509.9**

(22) Date of filing: **24.04.2008**

(54) **Edge detection in ultrasound images**

Kantenerkennung in Ultraschallbildern

Détection de bordure dans des images à ultrason

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **01.05.2007 US 915152 P**
**08.02.2008 US 28210**

(43) Date of publication of application:
**05.11.2008 Bulletin 2008/45**

(73) Proprietor: **Biosense Webster, Inc.**
**Diamond Bar, CA 91765 (US)**

(72) Inventors:
• **Zur, Dror**
**Haifa 34351 (IL)**
• **Mayzlish, Dov**
**Haifa 34332 (IL)**
• **Patt, Zafrir**
**Ramat Hasharon 47216 (IL)**

(74) Representative: **Small, Gary James et al**
**Carpmaels & Ransford**
**One Southampton Row**
**London**
**WC1B 5HA (GB)**

(56) References cited:
**EP-A- 1 030 187**

• JI L ET AL: "Loop-free snakes for highly irregular
object shapes" PATTERN RECOGNITION
LETTERS, ELSEVIER, AMSTERDAM, NL, vol. 23,
no. 5, 1 March 2002 (2002-03-01), pages 579-591,
XP004336094 ISSN: 0167-8655
• NOBLE J A ET AL: "Ultrasound image
segmentation: a survey" IEEE TRANSACTIONS
ON MEDICAL IMAGING, IEEE SERVICE CENTER,
PISCATAWAY, NJ, US, vol. 25, no. 8, 1 August
2006 (2006-08-01), pages 987-1010, XP008085509
ISSN: 0278-0062

Description

BACKGROUND OF THE INVENTION

Field of the Invention

This invention relates to medical imaging. More particularly, this invention relates to improvements in edge detection of intrabody structures in ultrasound images.
European patent publication no. EP 1030187 discloses an ultrasound segmentation method comprising the step of automated initial contour identification.
Ji L. et al in "Loop free snakes for highly irregular objects", published in Pattern Recognition Letters, El-sevier, Amsterdam, vol. 23, no.5 pages 579-591, discloses loop-free snakes that can remove all self-loops during their evolution.

Description of the Related Art

Ultrasound imaging is now well established as a modality for imaging structures in the body, such as the heart. For example, U.S. Patent No. 6,066,096
describes an imaging probe for volumetric intraluminal ultrasound imaging. The probe, configured to be placed inside a patient's body, includes an elongated body having proximal and distal ends. An ultrasonic transducer phased array is connected to and positioned on the distal end of the elongated body. The ultrasonic transducer phased array is positioned to emit and receive ultrasonic energy for volumetric forward scanning from the distal end of the elongated body. The ultrasonic transducer phased array includes a plurality of sites occupied by ultrasonic transducer elements.
Segmentation of ultrasound images in order to find 3-dimensional contours remains a difficult task, which generally requires substantial user involvement.

SUMMARY OF THE INVENTION

Embodiments of the present invention improve edge detection in 2-dimensional image data, e.g., ultrasound image data, that may be carried out automatically with minimal user involvement. The methods of edge detection in accordance with these embodiments are carried out nearly automatically, using an image processing technique that results in generation of a segmented edge contour, which may then be used in 3-dimensional reconstruction and segmentation.
An embodiment of the invention provides a computer-assisted method for defining structures on images, which is carried out by acquiring an image of a target structure, establishing a seed point within the structure on the image, detecting an edge in the image so as to generate a partially processed image having a computed edge indicated thereon, extending a plurality of rays radially from the seed point to intersect the computed edge at respective intersection points, and connecting the intersection points to form an initial closed contour in which respective segments connect neighboring intersection points. The method is further carried out by computing deforming force gradients in an area of interest of the image, deforming the closed contour responsively to the deforming force gradients to define a deformed closed contour, and deleting segments from the deformed closed contour that meet a predefined undesirability criterion.
One aspect of the method includes computing internal forces that oppose the deforming force gradients, wherein the closed contour is deformed responsively to a resolution of the internal forces and the deforming force gradients.
According to one aspect of the method, the image is an ultrasound image.
Another aspect of the method includes smoothing the image prior to detecting the edge.
According to a further aspect of the method, detecting the edge is performed by Canny edge detection.
According to yet another aspect of the method, the rays have an angular resolution not exceeding 5°.
Still another aspect of the method includes shrinking the closed contour toward the seed point.
An additional aspect of the method includes computing intensity gradients at respective edge points of the deformed closed contour. An edge segment whose intensity gradients are less than a predefined segmentation threshold meet the undesirability criterion.
According to one aspect of the method, the undesirability criterion includes a segment having a fold therein.
Other embodiments of the invention provide computer software product and apparatus for carrying out the above-described method.

BRIEF DESCRIPTION OF THE DRAWINGS

For a better understanding of the present invention, reference is made to the detailed description of the invention, by way of example, which is to be read in conjunction with the following drawings, wherein like elements are given like reference numerals, and wherein:

Fig. 1 is pictorially illustrates a system for obtaining and processing images in accordance with a disclosed embodiment of the invention;

Fig. 2 is a flow chart of a method of edge detection in an image, in accordance with a disclosed embodiment of the invention;

Fig. 3 is a 2-dimensional ultrasound image of a portion of a heart, which is suitable for image processing in accordance with the prior art;

Fig. 4 illustrates Canny edge detection performed on the image shown in Fig. 3, in which an initial edge has been determined in accordance with a disclosed embodiment of the invention;

Fig. 5 illustrates the image shown in Fig. 4, in which a series of rays radiate from a seed point, in accordance with a disclosed embodiment of the invention;

Fig. 6 illustrates a closed contour on the image Fig. 5, in accordance with a disclosed embodiment of the invention; and

Fig. 7 is a series of diagrams illustrating gap deletion in an image in accordance with a disclosed embodiment of the invention.

## DETAILED DESCRIPTION OF THE INVENTION

In the following description, numerous specific details are set forth in order to provide a thorough understanding of the present invention. It will be apparent to one skilled in the art, however, that the present invention may be practiced without these specific details. In other instances, well-known circuits, control logic and the details of computer program instructions for conventional algorithms and processes have not been shown in detail in order not to obscure the present invention unnecessarily.

Software programming code, which embodies aspects of the present invention, is typically maintained in permanent storage, such as a computer readable medium. In a client/server environment, such software programming code may be stored on a client or a server. The software programming code may be embodied on any of a variety of known tangible media for use with a data processing system, such as a diskette, or hard drive, or CD-ROM. The code may be distributed on such media, or may be distributed to users from the memory or storage of one computer system over a network of some type to storage devices on other computer systems for use by users of such other systems.

### System Overview

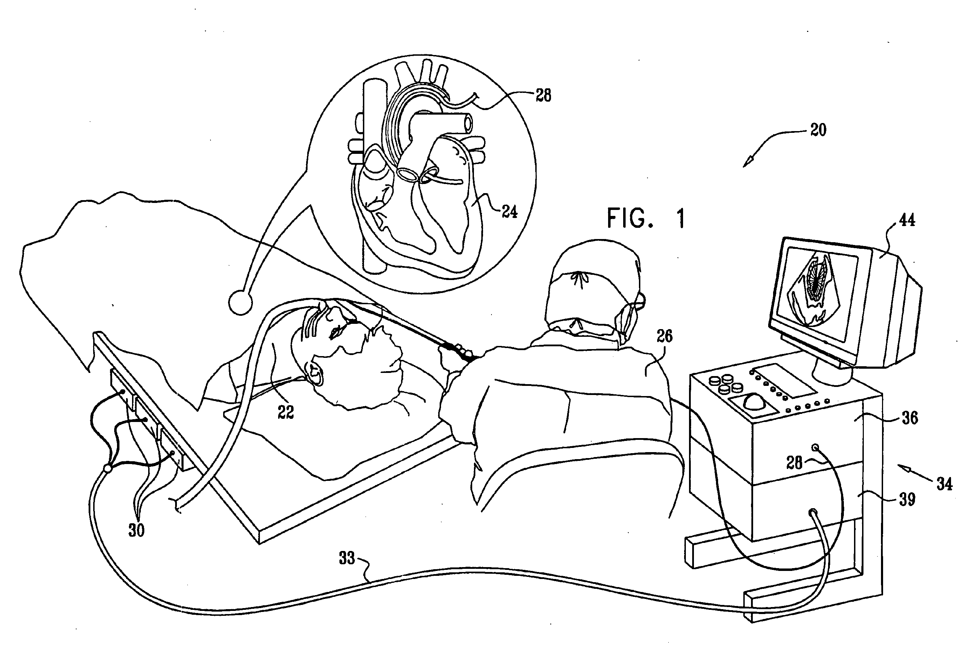

Turning now to the drawings, reference is initially made to Fig. 1, which is an illustration of a system 20 for ultrasound imaging and optionally for facilitating diagnostic and therapeutic procedures in a living patient in accordance with a disclosed embodiment of the invention. As shown in Fig. 1 a catheterization of the heart of a patient is being undertaken. This is exemplary, and the system 20 may be used for diverse procedures involving many organs of the body. Alternatively, ultrasound images may be acquired noninvasively, using conventional imaging equipment. The system comprises a catheter 28, which is percutaneously inserted by a physician into the body, here into a chamber or vascular structure of the heart.

The system 20 typically comprises a positioning subsystem that measures 3-dimensional location information and orientation coordinates of the catheter 28 with up to six degrees of freedom. Throughout this patent application, the term "location" refers to the spatial coordinates of the catheter, and the term "orientation" refers to its angular coordinates. The term "position" refers to the full positional information of the catheter, comprising both location and orientation coordinates. However, it is possible to practice the imaging procedures disclosed herein without recourse to the positioning subsystem. Indeed, in some embodiments, the positioning subsystem may be omitted.

In one embodiment, the positioning subsystem comprises a magnetic position tracking system that determines the position and orientation of the catheter 28. The positioning subsystem generates magnetic fields in a predefined working volume its vicinity, and senses these fields using one or more position sensors at the catheter. The positioning subsystem typically comprises a set of external radiators, such as field generating coils 30, which are located in fixed, known positions external to the patient. The coils 30 generate fields, typically electromagnetic fields, in the vicinity of the heart 24.

In an alternative embodiment, a radiator in the catheter, such as a coil, generates electromagnetic fields, which are received by sensors (not shown) outside the patient's body.

The position sensor transmits, in response to the sensed fields, position-related electrical signals over a cable 33 running through the catheter to a console 34. Alternatively, the position sensor may transmit signals to the console 34 over a wireless link. The console 34 comprises a positioning processor 36 that calculates the location and orientation of the catheter 28 based on the signals sent by a location sensor in the catheter (not shown). The positioning processor 36 typically receives, amplifies, filters, digitizes, and otherwise processes signals from the catheter 28. Images produced by the system 20 are displayed on a monitor 44.

For ultrasound image generation, the system 20 may employ the catheters disclosed in U.S. Patent Nos. 6,716,166 and 6,773,402. in order to acquire ultrasound images for display in near realtime. Ultrasound images may be acquired or displayed concurrently with an image or representation of the position of a deployment catheter in the same or different sessions, and in many different combinations. Such catheters have acoustic transducers that are adapted for emitting sound waves, and receiving reflections from echogenic interfaces in the heart. The reflections are then analyzed to construct two-dimensional and three-dimensional images of the heart.

The system 20 comprises an ultrasound driver 39 that drives the ultrasound transducers of the catheter 28 when it functions as an ultrasound imaging catheter. One example of a suitable ultrasound driver that can be used for this purpose is an AN2300™ ultrasound system produced by Analogic Corporation, 8 Centennial Drive, Peabody, MA 01960. The ultrasound driver 39 may support different imaging modes, such as B-mode, M-mode, CW Doppler and color flow Doppler, as are known in the art.

Image processing in the system 20 is carried out by a computer, which can be a general purpose computer, or a specialized device. The computer's processor accesses a memory that stores image data describing an image of a target structure, and stores executable objects including edge detection and smoothing programs. The operator can interact with the image processing phases via a graphical user interface on the monitor 44. The system 20 may be realized as the CARTO™ XP EP Navigation System version V9 (or higher) incorporating the SOUNDSTAR™ 3-dimensional diagnostic ultrasound catheter, both available from Biosense-Webster, Inc., 3333 Diamond Canyon Road, Diamond Bar, CA 91765.

**Operation**

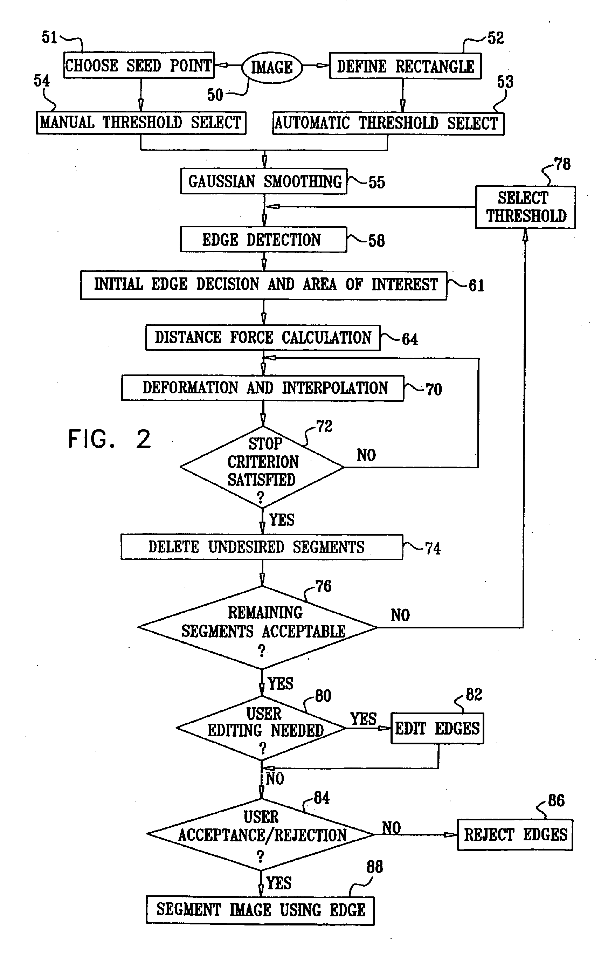

Reference is now made to Fig. 2, which is a flow chart of a method of edge detection of an image in accordance with a disclosed embodiment of the invention. At initial step 50, one or more grayscale ultrasound images of a target intrabody structure are acquired, for example using the system 20 (Fig. 1). The remainder of initial step 50 is performed interactively by an operator. The operator identifies an ultrasound image to which edge detection procedures are to be applied on the target structure. At step 51, the operator tentatively indicates a structure of interest, typically a cavity or chamber, to which image processing is to be applied. The operator marks a "seed point" at the center of the structure or cavity. The purpose of the seed point will become evident from the disclosure below. Manual or automatic threshold detection is elected by the operator.

When manual threshold detection is elected at step 51, then in step 54 a threshold is chosen by the operator. This involves the operator's judgment of the characteristics of the image, for example noise, and the degree of edge definition that appears on the image.

In an alternative sequence beginning at step 52, the operator may define a rectangle that is fully included within the target structure, e.g., within a cavity or an anatomic structure that is more sonolucent than its surroundings. The rectangle should contain the noisiest regions within the cavity, unless the noisy regions are "brighter" on the display than the edged area itself, in which case they should be excluded if possible. It is only necessary that most of the edge perimeter not be blocked from a view from the center of the rectangle by interposition of such noisy regions. When this alternative is elected, automatic threshold determination is applied at step 53. The geometric center of the rectangle becomes the seed point, and noise within the region defined by the rectangle is used to determine the threshold to be used automatically. Details of this procedure are described below. In general, relatively "noisy" images require higher thresholds than images having sharp contrasts.

At step 55, the chosen image is smoothed using a Gaussian smoothing operator, in order to reduce noise in the image prior to edge detection. Gaussian smoothing is essentially smoothing of image intensities using a mask defined by a 2-dimensional Gaussian function. This procedure is well known in the art. The smoothing may be done by convolving the image by a 7-by-7 bit mask, which contains a sample of a 2-dimensional = 3. Steps 53, 54 may be performed before Gaussian smoothing at step 55 as shown in Fig. 1. Alternatively, step 55 may precede steps 53, 54.

Next, at step 58, A program is executed that produces an initial binary edge map. The program employs Canny edge detection, applying the threshold determined in step 53 or step 54. In both steps 53, 54 the threshold is set at a level in which the Canny edge detection routine will fail to find edges inside the rectangle or in the region of the seed point as the case may be. Canny edge detection is well known, and is described in the document A Computational Approach to Edge Detection, F. J. Canny, IEEE Trans PAMI, 8(6):679-698, 1986. Intensity gradients are computed at each pixel in the detected edges, as described in the above-noted Canny document. The use of the intensity gradients is described below in the section entitled "Deletion of Undesired Segments".

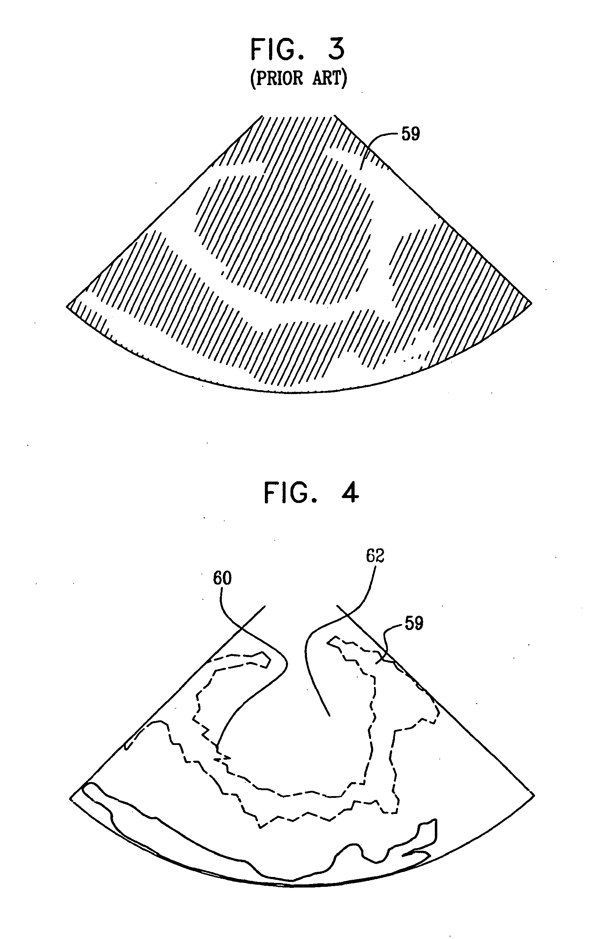

Reference is now made to Fig. 3, which is a conventional 2-dimensional ultrasound image of a portion of a heart, showing a solid mural region 59, which consists of solid tissue, which is suitable for image processing in accordance with a disclosed embodiment of the invention. Reference is now made to Fig. 4, which displays the result of Canny edge detection performed on the image shown in Fig. 3, in which an initial edge 60 has been determined in accordance with a disclosed embodiment of the invention. The edge 60 corresponds to an anatomical interface between the mural region 59, corresponding to myocardium, and an internal region 62 corresponding to the interior of a cardiac chamber, which in the common image plane of Fig. 3 and Fig. 4, is partially enclosed by the mural region 59. In this image, the threshold used in the Canny edge detection procedure could be determined automatically or manually, depending on whether step 53, or step 54 (Fig. 2) was elected.

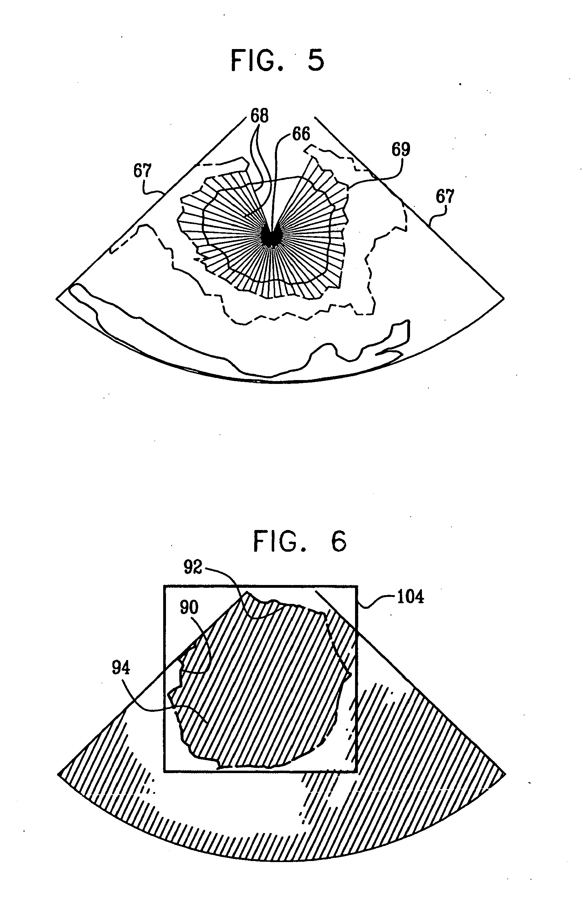

Reverting to Fig. 2, at step 58, an edge or border to be determined, typically the inner edge of a cavity, is located by extending rays outward from the seed point until the rays intersect the edge in the edge map. An angular resolution not exceeding 5° between rays is satisfactory. Reference is now made to Fig. 5, which illustrates the image shown in Fig. 4, in which a series of rays 68 radiate from a seed point 66 to intersect an edge 69, in accordance with a disclosed embodiment of the invention.

Referring again to Fig. 2, at step 61, neighboring intersection points (according to the angular order of their respective rays) as determined in step 58 are automatically connected to create a closed contour. Alternatively, the edges can be connected manually by the operator. In order to ensure that the edge is inside the cavity, the closed edge is shrunk by 20% toward the seed point. The details of establishing the closed contour are described in further detail below under the heading "Initial Contour". The borders of the enclosed space surrounded by the shrunken edge are referred to as the "initial edge".

Step 61 continues by defining a rectangular area of interest, includes a structure of interest, typically a cavity, and its surrounding edges. Reference is now made to Fig. 6, which illustrates an image of the structure shown in Fig. 4 and Fig. 5. A closed contour 92 has been drawn in accordance with a disclosed embodiment of the invention, enclosing a chamber 94 of interest, corresponding to the connection operation described with respect to step 61 (Fig. 2). An optional rectangular area of interest 104 is shown, which encompasses the chamber 94, its edge 90, and indeed, the entire closed contour 92. Outer rays 67 of the ultrasound fan are shown. Area of interest 104 is used to save computational effort by processing only the relevant part of the image.

Referring again to Fig. 2, next, at step 64, a distance transform is computed for each pixel. In embodiments in which the area of interest 104 (Fig. 6) is employed, the computation is limited to the area of interest 104. Otherwise, the computation is applied to the entire image, or at least the area within the closed contour 92. This transform maps the distances from the pixels to the nearest point on the edges on the edge map in the area of interest. The result is used to calculate a distance gradient for each pixel. The details of the calculation are presented below under the heading "Distance Transformation".

Next, at step 70, the deformation of the initial edge is performed according to the theory of deformable models (sometimes also called snakes), using parametric formulation with dynamic force. Deformable models are known from the document, "Image Segmentation Using Deformable Models," Chenyang Xu, Dzung Pham, and Jerry Prince, in Handbook of Medical Imaging -- Volume 2: Medical Image Processing and Analysis, pp. 129-174, SPIE Press, May 2000,. A summary of the computation is given below under the heading "Deformation and Interpolation".

Control now proceeds to decision step 72, where it is determined if a stop criterion exists. Deformation of the edge stops when the derivative at the right side of Equation 2 (described below) becomes zero. Alternatively, the algorithm may halt after a predefined number of iterations, or until no inflation of the edge is observed, whichever occurs first. In the latter case, the number of pixels contained by the edge is not growing, indicating a steady state.

If the determination at decision step 72 is negative, then control returns to step 70.

If the determination at decision step 72 is affirmative, then control proceeds to step 74, where undesired or disqualified segments are deleted. There are several types of undesired segments. Details of step 74 are given below under the heading "Deletion of Undesired Segments".

Control now proceeds to decision step 76, where it is determined if the segments remaining after deletion of undesired segments in step 74 produce an acceptable contour. This determination is normally made by an operator. The contour may be corrected automatically in order to correctly remove spurious segments and gaps. Alternatively, the contour may be corrected manually.

If the determination at decision step 76 is negative, then control proceeds to step 78. When performed interactively, the user may assist the process, edit the result, and vary parameters of the algorithm. At step 78, the user changes the edge detection threshold. Control returns to step 58, where edge detection is repeated with the new threshold.

If the determination at decision step 76 is affirmative, then control proceeds to decision step 80, where it is determined by the operator if supplemental interactive correction of the automatic edges is required.

If the determination at decision step 80 is affirmative, then control proceeds to step 82. The edges are edited in manual mode. In manual mode, the user has the option to correct the edge manually on the image using a graphic pencil and eraser. With this option, it is possible for the operator to correct or delete segments that were improperly retained in during gap deletion in step 74.

After completion of step 82, or if the determination at decision step 80 is negative, control proceeds to decision step 84. This is a quality control step, in which a determination is made whether the result thus far achieved is acceptable.

If the determination at decision step 84 is negative, then control proceeds to final step 86. The contour is rejected.

If the determination at decision step 84 is affirmative, then control proceeds to final step 88. The contour has now been segmented and is accepted.

**Initial Contour**

A closed contour (step 61, Fig. 2) is generated as follows: Starting with the intersection point nearest the seed point, the distances between the remaining intersection points and the seed point are determined successively. If the difference of the distances from the seed point between two successive intersection points exceeds a predetermined threshold then the more distant intersection point is ignored. Fifteen pixels is a suitable value for the threshold. Then, starting from the nearest intersection point the points are connected using linear interpolation to create initial edge segments. Next, long sequences exceeding a predefined length, currently 35 degrees or 7 rays, of canceled intersection points are assumed to be wrongly canceled. The scanning algorithm is repeated for these sequences, using a lower threshold, currently 2/3 of the previous threshold. If previously ignored intersection points are now approved, they are connected to create additional segments. This procedure is repeated, until there are no canceled sequences that are larger then a predefined value. Then, all gaps between the approved segments are connected to form the closed edge contour. Finally, in order to ensure that the edge is inside the cavity, the enclosed space is reduced in volume by 20%, retaining the seed point as the geometric center of the contour.

**Distance Transformation**

The distance gradients correspond to deforming "forces" on the edges, as given by Equation 1, and are sometimes referred to as "deforming force gradients". The terms "force" and "forces" are used arbitrarily herein to indicate the magnitude of the influence of the gradients on the contours of structures on the image. Otherwise, the term has no physical meaning with respect to the images being processed.

$$DF(x,y) = \nabla DT(x,y) = \left( {}_{x}DT(x,y), \ {}_{y}DT(x,y) \right)$$

$$(1)$$

The calculation is done in the following way: First the Euclidian distance is calculated between each pixel in the image and the closest pixel on the initial edge map, that is the closed contour defined in step 61. This phase is referred to as a "distance transform" and results in a distance map, which includes the minimum distance for each pixel. Then, for each pixel, a gradient is calculated over the distance map, which was created in the previous phase:

The gradients are then iteratively applied, as an "external force", to deform the closed contour 92 (Fig. 6) until the best match between the contour and a subset of the edges in the edge map (usually an inner subset is found. This is typically a subset of edges closest to the seed point.

**Deformation and Interpolation**

The deformation computation is shown in Equation 2:

$$\gamma \frac{X}{t} = F_{int}(X) + F_{ext}(X) \qquad (2),$$

where X is the collection of edge points (starting from the initial edge) and ge is the damping coefficient. $F_{int}(X)$ are analogous to internal physical forces, which are activated on edge points, e.g., of the closed contour 92 (Fig. 6), calculated using Equation 3:

$$F_{int} = -\frac{}{s}(\alpha \frac{X}{s}) - \frac{2}{s^2}(\beta \frac{^2 X}{s^2}) \qquad (3).$$

Here, $\alpha$ is a tension parameter, which discourages stretching and makes the deformed edge behave as an elastic string. $\beta$ is a rigidity parameter, which discourages bending and makes the deformed edge behave like a rigid rod. The internal forces $F_{int}(X)$ oppose the external force, which is the gradient that was calculated in step 64 (Fig. 2). The gradient has the general effect of locally attracting and deforming the edge. The actual deformation is performed in accordance with a resolution of the internal forces and the gradients. Every five iterations, a linear interpolation is performed on the edge points in order to add new edge points and to eliminate any local dilations that result from too sparse a distribution of the edge points.

**Deletion of Undesired Segments**

Edge segments meeting criteria of undesirability are deleted. A first category includes edge segments that reach the external rays of the ultrasound fan on an ultrasound image. These are deleted. Since the coordinate of the external rays are transferred to the algorithm this is done simply by adding to the edges map two illusory edges parallel and very close to the rays outside of the fan. Every edge segment that crossed the rays and was attached to the illusory edges is deleted.

Another type of undesired segment is a folded or looped segment. Such segments are detected when two non-successive points along an edge are located very close to each other. For purposes of this procedure, two points lying within 2 pixels of each other on the image, but which are at least 5 pixels apart when measured along the edge, are considered to constitute a loop or fold. In such case, all the points between these two points are deleted.

Finally, a procedure that results in deletion of areas representing anatomic gaps is applied to the image. Only those points on the closed contour having a sufficient signal-to-noise ratio are retained. For every pixel in the final edge, the Canny edge detection program (step 58, Fig. 2) calculates an intensity gradient in which the smoothed original image is projected on a line perpendicular to the edge at that point. The resulting value is a measure for the significance of the edge at that point. To overcome noise influence, the sequence (according to the order of the points of the edge) of significance values is smoothed. The curve is then segmented according to a predefined "segmentation threshold". Edge points that have a significance value exceeding the threshold are spared; the others are deleted. Gaps are then bridged by reconnecting new neighboring intersection points to reform the closed contour. A suitable segmentation threshold is 40% of the maximum value.

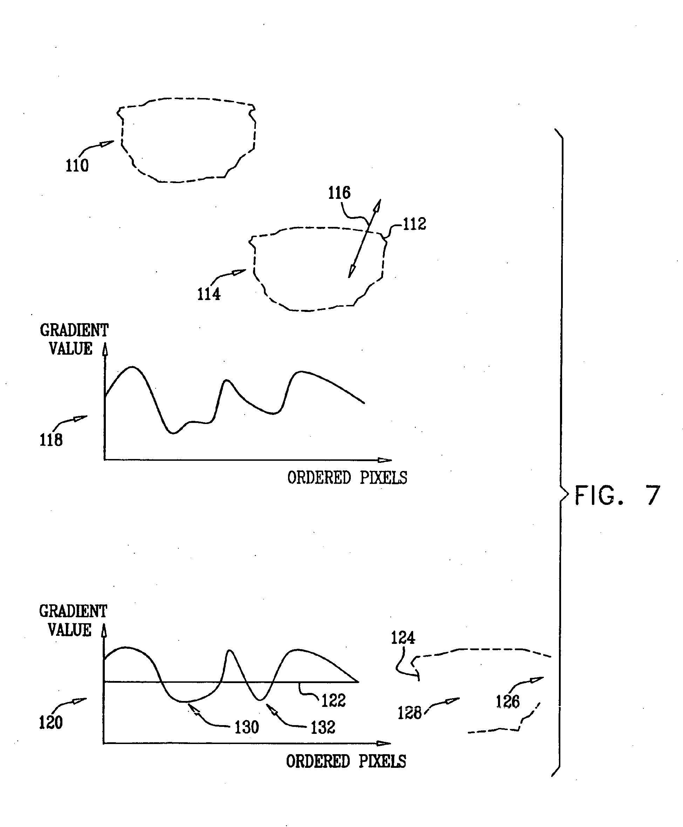

Reference is now made to Fig. 7, which is a series of diagrams, illustrating gap deletion in an image in accordance with a disclosed embodiment of the invention. At the top of Fig. 7, a view 110, a closed contour of a target structure 112 is of interest.

In view 114, intensity gradients are calculated normal to the edge of each point on the contour of the target structure 112. A representative intensity gradient is indicated by an arrow 116.

Graph 118 is a plot of the intensity gradients against ordered pixels. In graph 120, a smoothing operation has been applied to the data shown in graph 118. Smoothing has the effect of eliminating aberrant local values. A threshold 122 is shown. Only those points having intensity gradients exceeding the threshold 122 are retained in the final result, which is represented by contour 124. Excluded points are indicated as gaps 126, 128, which correspond to intervals 130, 132.

The scope of the present invention is defined by the appended claims.

**Claims**

1.  A computer-assisted method for defining structures on images, comprising the steps of:

acquiring (50) an image of a target structure for edge detection thereof;
establishing (51) a seed point (66) within said structure on said image based on user input;
detecting (58) edges (69) in said image so as to generate a partially processed image of said structure having a computed edge indicated thereon;
extending a plurality of rays (68) radially from said seed point to intersect said computed edge at respective intersection points;

connecting (61) said intersection points to form an initial closed contour (92) having respective segments connecting neighboring ones of said intersection points;

computing (64), for each pixel in an area of interest of said image (104), a distance transform from said edges;

computing deforming forces based on gradients of said distance transform;

deforming (70) said closed contour responsively to said deforming forces to define a deformed closed contour; and

deleting (74) segments from said deformed closed contour that meet a predefined undesirability criterion.

2. The method according to claim 1, further comprising the step of computing internal forces that oppose said deforming forces, wherein said step of deforming is performed responsively to a resolution of said internal forces and said deforming forces.

3. The method according to claim 1, wherein said image is an ultrasound image.

4. The method according to claim 1, further comprising the steps of smoothing (55) said image prior to detecting said edge.

5. The method according to claim 1, wherein detecting said edge comprises Canny edge detection.

6. The method according to claim 1, wherein said rays have an angular resolution not exceeding 5°.

7. The method according to claim 1, further comprising the step of shrinking said closed contour toward said seed point before deforming said closed contour.

8. The method according to claim 1, further comprising the step of computing intensity gradients at respective edge points of said deformed closed contour, said undesirability criterion comprising a segment wherein said intensity gradients of said edge points thereof are less than a predefined segmentation threshold.

9. The method according to claim 1, wherein said undesirability criterion comprises a segment having a loop therein.

10. A computer software product for defining structures on images, including a computer storage medium in which computer program instructions are stored, which instructions, when executed by a computer, cause the computer to:

accept data describing an image of a target structure for edge detection thereof;

establish a seed point (66) within said structure on said image based on user input;

detect edges with an edge detection program in said image so as to generate a partially processed image of said structure having a computed edge (69) indicated thereon;

extend a plurality of rays (68) radially from said seed point to intersect said computed edge at respective intersection points;

connect said intersection points to form an initial closed contour (92) having respective segments connecting neighboring ones of said intersection points; the instructions further causing the computer to compute, for each pixel in an area of interest of said image (104), a distance transform from said edges compute deforming forces at respective locations based on gradients of said distance transform on said closed contour;

deform said closed contour responsively to said deforming forces to define a deformed closed contour; and

delete segments on said deformed closed contour that meet a predefined undesirability criterion.

11. The computer software product according to claim 10, wherein said computer is further instructed to compute internal forces that oppose said deforming forces, to deform said closed contour responsively to a resolution of said internal forces and said deforming forces.

12. The computer software product according to claim 10, wherein said image is an ultrasound image.

13. The computer software product according to claim 10, wherein said computer is further instructed to execute a smoothing program to smooth said image prior to executing said edge detection program.

14. The computer software product according to claim 10, wherein said edge detection program comprises Canny edge detection.

**15.** The computer software product according to claim 10, wherein said rays have an angular resolution not exceeding 5°.

**16.** The computer software product according to claim 10, wherein said computer is further instructed to shrink said closed contour toward said seed point before deforming said closed contour.

**17.** The computer software product according to claim 10, wherein said computer is further instructed to calculate intensity gradients at respective edge points of said deformed closed contour, said undesirability criterion comprising a segment wherein said intensity gradients of said edge points thereof are less than a predefined segmentation threshold.

**18.** The computer software product according to claim 10, wherein said undesirability criterion comprises a segment having a loop therein.

**19.** A system (20) for defining structures on images, comprising:

a display;
a memory for storing data describing an image of a target structure, and storing executable objects comprising an edge detection program; and
a processor linked to said memory and, said processor operative to process said data, to establish a seed point (66) within said structure on said image based on user input, to detect edges with said edge detection program in said image so as to generate a partially processed image of said structure having a computed edge (69) indicated thereon, to extend a plurality of rays (68) radially from said seed point to intersect said computed edge at respective intersection points, to connect said intersection points to form an initial closed contour having respective segments connecting neighboring ones of said intersection points,
said processor being operative to compute, for each pixel in an area of interest of said image (104), a distance transform from said edges to compute deforming forces based on gradients of said distance transform at respective locations on said closed contour, to deform said closed contour responsively to said deforming forces to define a deformed closed contour, to delete segments on said deformed closed contour that meet a predefined undesirability criterion to define a processed image, and to present said processed image on said display.

**20.** The system according to claim 19, wherein said image is an ultrasound image.

**21.** The system according to claim 19, wherein said processor is operative to calculate intensity gradients at respective edge points of said deformed closed contour, said undesirability criterion comprising a segment wherein said intensity gradients of said edge points thereof are less than a predefined segmentation threshold.

**22.** The system according to claim 19, wherein said undesirability criterion comprises a segment having a loop therein.

**Patentansprüche**

**1.** Computerunterstütztes Verfahren zum Definieren von Strukturen auf Bildern, das die Schritte aufweist:

Erlangen (50) eines Bildes einer Zielstruktur für deren Kantenerfassung;
Einrichten (51) eines Keimpunktes (66) innerhalb der Struktur auf dem Bild basierend auf der Eingabe eines Benutzers;
Erfassen (58) von Kanten (69) in dem Bild, um so ein teilweise verarbeitetes Bild der Struktur zu erzeugen, das eine berechnete Kante hat, die darauf angegeben ist;
Erstrecken einer Vielzahl von Strahlen (68) radial von dem Keimpunkt, die die berechnete Kante an jeweiligen Schnittpunkten schneiden;
Verbinden (61) der Schnittpunkte, um eine anfängliche geschlossene Kontur (92) mit jeweiligen Segmenten zu bilden, die benachbarte der Schnittpunkte verbinden;
Berechnen, für jeden Pixel in einem interessierenden Gebiet des Bildes (104), einer Distanztransformation von den Kanten;
Berechnen deformierender Kräfte basierend auf Gradienten der Distanztransformation;
Deformieren (70) der geschlossenen Kontur ansprechend auf die deformierenden Kräfte, um eine deformierte geschlossene Kontur zu definieren; und
Löschen (74) von Segmenten aus der deformierten geschlossenen Kontur, die ein vorab definiertes Unerwünschtheitskriterium erfüllen.

2. Verfahren nach Anspruch 1, das weiter den Schritt des Berechnens innerer Kräfte, die den deformierenden Kräften entgegen wirken, aufweist, wobei der Schritt des Deformierens ansprechend auf eine Auflösung der inneren Kräfte und der deformierenden Kräfte durchgeführt wird.

3. Verfahren nach Anspruch 1, bei dem das Bild ein Ultraschallbild ist.

4. Verfahren nach Anspruch 1, weiter mit den Schritten des Glättens (55) des Bildes vor dem Erfassen der Kante.

5. Verfahren nach Anspruch 1, bei dem das Erfassen der Kante eine Kantendetektion nach Canny aufweist.

6. Verfahren nach Anspruch 1, bei dem die Strahlen eine Winkelauflösung haben, die 5° nicht überschreitet.

7. Verfahren nach Anspruch 1, das weiter den Schritt des Schrumpfens der geschlossenen Kontur auf den Keimpunkt zu, bevor die geschlossene Struktur deformiert wird, aufweist.

8. Verfahren nach Anspruch 1, das weiter den Schritt des Berechnens von Intensitätsgradienten an jeweiligen Kantenpunkten der deformierten geschlossenen Kontur aufweist, wobei das Unerwünschtheitskriterium ein Segment aufweist, in dem die Intensitätsgradienten der Kantenpunkte kleiner sind als ein vorab definierter Segmentierungsschwellenwert.

9. Verfahren nach Anspruch 1, bei dem das Unerwünschtheitskriterium ein Segment mit einer Schleife darin aufweist.

10. Computersoftwareprodukt zum Definieren von Strukturen auf Bildern, das ein Computerspeichermedium umfasst, in dem Computerprogrammbefehle gespeichert sind, wobei die Befehle, wenn sie von einem Computer ausgeführt werden, bewirken, dass der Computer:

 Daten akzeptiert, die ein Bild einer Zielstruktur für dessen Kantenerfassung beschreiben;
 einen Keimpunkt (66) innerhalb der Struktur auf dem Bild, basierend auf der Eingabe eines Benutzers, einrichtet;
 Kanten mit einem Kantenerfassungsprogramm in dem Bild erfasst, um so ein teilweise verarbeitetes Bild der Struktur zu erzeugen, das eine berechnete Kante (69) hat, die darauf angegeben ist;
 eine Vielzahl von Strahlen (68) radial von dem Keimpunkt erstreckt, die die berechnete Kante an jeweiligen Schnittpunkten schneiden;
 die Schnittpunkte verbindet, um eine anfängliche geschlossene Kontur (92) zu bilden, die jeweilige Segmente hat, welche benachbarte der Schnittpunkte verbinden;
 wobei die Befehle weiter bewirken, dass der Computer
 für jeden Pixel in einem interessierenden Gebiet des Bildes (104) eine Distanztransformation von den Kanten berechnet, deformierende Kräfte an jeweiligen Orten basierend auf Gradienten der Distanztransformation auf der geschlossen Kontur berechnet,
 die geschlossene Kontur ansprechend auf die deformierenden Kräfte deformiert, um eine deformierte geschlossene Kontur zu definieren; und
 Segmente in der deformierten geschlossenen Kontur löscht, die ein vorab definiertes Unerwünschtheitskriterium erfüllen.

11. Computersoftwareprodukt nach Anspruch 10, bei dem dem Computer weiter befohlen wird, innere Kräfte zu berechnen, die den deformierenden Kräften entgegengesetzt sind, um die geschlossene Kontur ansprechend auf eine Auflösung der inneren Kräfte und der deformierenden Kräfte zu deformieren.

12. Computersoftwareprodukt nach Anspruch 10, bei dem das Bild ein Ultraschallbild ist.

13. Computersoftwareprodukt nach Anspruch 10, bei dem dem Computer weiter befohlen wird, ein Glättungsprogramm auszuführen, um das Bild vor dem Ausführen des Kantenerfassungsprogrammes zu glätten.

14. Computersoftwareprodukt nach Anspruch 10, bei dem das Kantenerfassungsprogramm eine Kantendetektion nach Canny aufweist.

15. Computersoftwareprodukt nach Anspruch 10, bei dem die Strahlen eine Winkelauflösung haben, die 5° nicht überschreitet.

**16.** Computersoftwareprodukt nach Anspruch 10, bei dem dem Computer weiter befohlen wird, die geschlossene Kontur auf den Keimpunkt zu zu schrumpfen, bevor die geschlossene Kontur deformiert wird.

**17.** Computersoftwareprodukt nach Anspruch 10, bei dem dem Computer weiter befohlen wird, Intensitätsgradienten an jeweiligen Kantenpunkten der deformierten geschlossenen Kontur zu berechnen, wobei das Unerwünschtheitskriterium ein Segment aufweist, in dem die Intensitätsgradienten der Kantenpunkte kleiner sind als ein vorab definierter Segmentierungsschwellenwert.

**18.** Computersoftwareprodukt nach Anspruch 10, bei dem das Unerwünschtheitskriterium ein Segment mit einer Schleife darin aufweist.

**19.** System (20) zum Definieren von Strukturen auf Bildern, das aufweist:

eine Anzeigeeinheit;
einen Speicher zum Speichern von Daten, die ein Bild einer Zielstruktur beschreiben, und zum Speichern ausführbarer Objekte, welche ein Kantenerfassungsprogramm aufweisen; und
einen Prozessor, der mit dem Speicher verknüpft ist und der, wobei der Prozessor so betrieben wird, dass er die Daten verarbeitet, einen Keimpunkt (66) innerhalb der Struktur auf dem Bild basierend auf der Eingabe eines Benutzers einrichtet, Kanten mit dem Kantenerfassungsprogramm in dem Bild erfasst, um so ein teilweise verarbeitetes Bild der Struktur, welche eine berechnete Kante (69) hat, die darauf angegeben ist, zu erzeugen, eine Vielzahl von Strahlen (68) radial von dem Keimpunkt erstreckt, die die berechnete Kante an jeweiligen Schnittpunkten schneiden, die Schnittpunkte verbindet, um eine anfängliche geschlossene Kontur mit jeweiligen Segmenten zu bilden, welche benachbarte der Schnittpunkte verbinden,
wobei der Prozessor so betrieben wird, dass er, für jeden Pixel in einem interessierenden Gebiet des Bildes (104) eine Distanztransformation von den Kanten berechnet, deformierende Kräfte basierend auf Gradienten der Distanztransformation an jeweiligen Orten auf der geschlossenen Kontur berechnet, die geschlossene Kontur ansprechend auf die deformierenden Kräfte deformiert, um eine deformierte geschlossene Kontur zu definieren, Segmente auf der deformierten geschlossenen Kontur löscht, die ein vorab definiertes Unerwünschtheitskriterium erfüllen, um ein verarbeitetes Bild zu definieren, und das verarbeitete Bild auf der Anzeigeeinheit darstellt.

**20.** System nach Anspruch 19, bei dem das Bild ein Ultraschallbild ist.

**21.** System nach Anspruch 19, bei dem der Prozessor so betrieben wird, dass er Intensitätsgradienten an jeweiligen Kantenpunkten der deformierten geschlossenen Kontur berechnet, wobei das Unerwünschtheitskriterium ein Segment aufweist, in dem die Intensitätsgradienten der Kantenpunkte kleiner sind als ein vorab definierter Segmentierungsschwellenwert.

**22.** System nach Anspruch 19, bei dem das Unerwünschtheitskriterium ein Segment mit einer Schleife darin aufweist.

**Revendications**

**1.** Procédé assisté par ordinateur destiné à définir des structures sur des images, comprenant les étapes consistant à :

■ acquérir (50) une image d'une structure cible afin de détecter un bord de celle-ci ;
■ établir (51) un point graine (66) à l'intérieur de ladite structure sur ladite image sur la base d'une entrée d'utilisateur ;
■ détecter (58) des bords (69) dans ladite image de manière à générer une image traitée en partie de ladite structure sur laquelle est indiqué un bord calculé ;
■ étendre de manière radiale une pluralité de rayons (68) à partir dudit point graine de manière à couper ledit bord calculé à des points d'intersection respectifs ;
■ relier (61) lesdits points d'intersection de manière à former un contour fermé initial (92) qui présente des segments respectifs qui relient lesdits points d'intersection voisins ;
■ calculer (64), pour chaque pixel dans une zone qui présente un intérêt de ladite image (104), une transformée de distance à partir desdits bords ;

■ calculer des forces de déformation sur la base de gradients de ladite transformée de distance ;
■ déformer (70) ledit contour fermé en réponse auxdites forces de déformation de manière à définir un contour fermé déformé ; et
■ supprimer (74) des segments dudit contour fermé déformé qui répondent à un critère de caractère indésirable prédéfini.

2. Procédé selon la revendication 1, comprenant en outre l'étape consistant à calculer des forces internes qui s'opposent auxdites forces de déformation, dans lequel ladite étape de déformation est exécutée en réponse à une résolution desdites forces internes et desdites forces de déformation.

3. Procédé selon la revendication 1, dans lequel ladite image est une image ultrasonore.

4. Procédé selon la revendication 1, comprenant en outre les étapes consistant à lisser (55) lesdistes images avant de détecter ledit bord.

5. Procédé selon la revendication 1, dans lequel la détection dudit bord comprend une détection de bord de Canny.

6. Procédé selon la revendication 1, dans lequel lesdits rayons présentent une résolution angulaire qui ne dépasse pas 5°.

7. Procédé selon la revendication 1, comprenant en outre l'étape consistant à rétrécir ledit contour fermé vers ledit point graine avant de déformer ledit contour fermé.

8. Procédé selon la revendication 1, comprenant en outre l'étape consistant à calculer des gradients d'intensité à des points de bord respectifs dudit contour fermé déformé, ledit critère de caractère indésirable comprenant un segment dans lequel lesdits gradients d'intensité desdits points de bord de celui-ci sont inférieurs à un seuil de segmentation prédéfini.

9. Procédé selon la revendication 1, dans lequel ledit critère de caractère indésirable comprend un segment qui présente à l'intérieur une boucle.

10. Produit de logiciel informatique destiné à définir des structures sur des images, comprenant un support de stockage informatique dans lequel sont stockées des instructions de programme informatique, lesquelles instructions, quand elles sont exécutées par un ordinateur, font que l'ordinateur :

■ reçoit des données qui décrivent une image d'une structure cible afin de détecter un bord de celle-ci ;
■ établit un point graine (66) à l'intérieur de ladite structure sur ladite image sur la base d'une entrée d'utilisateur ;
■ détecte des bords, à l'aide d'un programme de détection de bord, dans ladite image de manière à générer une image traitée en partie de ladite structure sur laquelle est indiqué un bord calculé (69) ;
■ étend de manière radiale une pluralité de rayons (68) à partir dudit point graine de manière à couper ledit bord calculé à des points d'intersection respectifs ;
■ relie lesdits points d'intersection de manière à former un contour fermé initial (92) qui présente des segments respectifs qui relient lesdits points d'intersection voisins ;
■ les instructions faisant en outre en sorte que l'ordinateur :

■ calcule, pour chaque pixel dans une zone qui présente un intérêt de ladite image (104), une transformée de distance à partir desdits bords ;
■ calcule des forces de déformation à des emplacements respectifs sur la base des gradients de ladite transformée de distance sur ledit contour fermé ;
■ déforme ledit contour fermé en réponse auxdites forces de déformation de manière à définir un contour fermé déformé ; et
■ supprime des segments sur ledit contour fermé déformé qui répondent à un critère de caractère indésirable prédéfini.

11. Produit de logiciel informatique selon la revendication 10, dans lequel ledit ordinateur est chargé en outre de calculer des forces internes qui s'opposent auxdites forces de déformation, de manière à déformer ledit contour fermé en réponse à une résolution desdites forces internes et desdites forces de déformation.

**12.** Produit de logiciel informatique selon la revendication 10, dans lequel ladite image est une image ultrasonore.

**13.** Produit de logiciel informatique selon la revendication 10, dans lequel ledit ordinateur est chargé en outre d'exécuter un programme de lissage de manière à lisser ladite image avant d'exécuter ledit programme de détection de bord.

**14.** Produit de logiciel informatique selon la revendication 10, dans lequel ledit programme de détection de bord comprend une détection de bord de Canny.

**15.** Produit de logiciel informatique selon la revendication 10, dans lequel lesdits rayons présentent une résolution angulaire qui ne dépasse pas 5°.

**16.** Produit de logiciel informatique selon la revendication 10, dans lequel ledit ordinateur est chargé en outre de rétrécir ledit contour fermé vers ledit point graine avant de déformer ledit contour fermé.

**17.** Produit de logiciel informatique selon la revendication 10, dans lequel ledit ordinateur est chargé en outre de calculer des gradients d'intensité à des points de bord respectifs dudit contour fermé déformé, ledit critère de caractère indésirable comprenant un segment dans lequel lesdits gradients d'intensité desdits points de bord de celui-ci sont inférieurs à un seuil de segmentation prédéfini.

**18.** Produit de logiciel informatique selon la revendication 10, dans lequel ledit critère de caractère indésirable comprend un segment qui présente à l'intérieur une boucle.

**19.** Système (20) destiné à définir des structures sur des images, comprenant :

■ un affichage ;
■ une mémoire destinée à stocker des données qui décrivent une image d'une structure cible, et à stocker des objets exécutables qui comprennent un programme de détection de bord ; et
■ un processeur relié à ladite mémoire et, ledit processeur étant opérationnel de manière à : traiter lesdites données, établir un point graine (66) l'intérieur de ladite structure sur ladite image sur la base d'une entrée d'utilisateur, détecter des bords à l'aide dudit programme de détection de bord dans ladite image afin de générer une image traitée en partie de ladite structure sur laquelle est indiqué un bord calculé (69), étendre de manière radiale une pluralité de rayons (68) à partir dudit point graine de façon à couper ledit bord calculé à des points d'intersection respectifs, relier lesdits points d'intersection de manière à former un contour fermé initial qui présente des segments respectifs qui relient lesdits points d'intersection voisins ;

ledit processeur étant opérationnel de manière à : calculer, pour chaque pixel dans une zone qui présente un intérêt de ladite image (104), une transformée de distance à partir desdits bords, calculer des forces de déformation sur la base des gradients de ladite transformée de distance à des endroits respectifs sur ledit contour fermé, déformer ledit contour fermé en réponse auxdites forces de déformation de façon à définir un contour fermé déformé, supprimer des segments sur ledit contour fermé déformé qui répondent à un critère de caractère indésirable prédéfini de manière à définir une image traitée, et présenter ladite image traitée sur ledit affichage.

**20.** Système selon la revendication 19, dans lequel ladite image est une image ultrasonore.

**21.** Système selon la revendication 19, dans lequel ledit processeur est opérationnel de manière à calculer des gradients d'intensité à des points de bord respectifs dudit contour fermé déformé, ledit critère de caractère indésirable comprenant un segment dans lequel lesdits gradients d'intensité desdits points de bord de celui-ci sont inférieurs à un seuil de segmentation prédéfini.

**22.** Système selon la revendication 19, dans lequel ledit critère de caractère indésirable comprend un segment qui présente à l'intérieur une boucle.

FIG. 1

FIG. 2

```
 51 ──┐                                                    ┌── 52
  ┌─────────────────┐      ╭─────────╮      ┌──────────────────┐
  │ CHOOSE SEED POINT│◄────┤  IMAGE  ├────►│ DEFINE RECTANGLE  │
  └─────────────────┘      ╰─────────╯      └──────────────────┘
 54 ┐        │            50─┘     │                    │        ┌ 53
  ┌──────────────────────┐   ┌──────────────────────────────┐
  │ MANUAL THRESHOLD SELECT│   │ AUTOMATIC THRESHOLD SELECT   │
  └──────────────────────┘   └──────────────────────────────┘
```

- CHOOSE SEED POINT — 51
- IMAGE — 50
- DEFINE RECTANGLE — 52
- MANUAL THRESHOLD SELECT — 54
- AUTOMATIC THRESHOLD SELECT — 53
- GAUSSIAN SMOOTHING — 55
- SELECT THRESHOLD — 78
- EDGE DETECTION — 58
- INITIAL EDGE DECISION AND AREA OF INTEREST — 61
- DISTANCE FORCE CALCULATION — 64
- DEFORMATION AND INTERPOLATION — 70
- STOP CRITERION SATISFIED ? — 72 — NO
- DELETE UNDESIRED SEGMENTS — 74
- REMAINING SEGMENTS ACCEPTABLE ? — 76 — NO
- USER EDITING NEEDED ? — 80 — YES — EDIT EDGES — 82
- USER ACCEPTANCE/REJECTION ? — 84 — NO — REJECT EDGES — 86
- SEGMENT IMAGE USING EDGE — 88

## FIG. 3
(PRIOR ART)

## FIG. 4

# FIG. 5

# FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1030187 A
- US 6066096 A
- US 6716166 B
- US 6773402 B

### Non-patent literature cited in the description

- Loop free snakes for highly irregular objects. **Ji L. et al.** Pattern Recognition Letters. El-sevier, vol. 23, 579-591
- **F. J. CANNY.** A Computational Approach to Edge Detection. *IEEE Trans PAMI,* 1986, vol. 8 (6), 679-698
- **CHENYANG XU ; DZUNG PHAM ; JERRY PRINCE.** Handbook of Medical Imaging -- Volume 2: Medical Image Processing and Analysis. SPIE Press, May 2000, vol. 2, 129-174